# EUROPEAN PATENT APPLICATION

(11) **EP 2 011 442 A1**
(43) Date of publication of application: **07.01.2009**
(21) Application number: 07111506.7
(22) Date of filing: 02.07.2007
(51) Int. Cl.: A61B 17/02

(54) **Pliers for separating two adjacent bone segments**

(71) Applicant: Berner Fachhochschule, Technik und Informatik (HTI), 2501 Biel (CH)
(72) Inventor: The designation of the inventor has not yet been filed
(74) Representative: Liebetanz, Michael

(57) **Abstract**

The manually usable pliers are used by a surgeon for separating two adjacent bone segments. The surgeon takes the handles (10, 11) which are connected with jaws (20, 21) through a fulcrum (30). The pliers furthermore comprise a sensor (70) for determining the distraction distance between said two bone segments and at least one additional sensor (80, 81) for determining the distraction force acting on such bone segments upon opening of the jaws through manually applicable forces on the handle portions (40, 41). The sensors (70, 80, 81) are electronic sensors. The measurement allows to directly calculate the stiffness of the adjacent bone segments.

## Description

### Field of the invention

The present invention relates to manually usable pliers for separating two adjacent bone segments according to the preamble of claim 1. Surgical instrument having this function are also known as distractors.

### Technical background of the invention

US 2001/0031969 A1 describes a distractor for separating adjacent vertebrae. The handle of the pliers comprises a spring opening the handle and thus closing the jaws of the distractor. The blades of the distractor can be exchanged following the needs of the surgeon. Each blade may comprise a blade post 62 to be fitted within a socket of the jaws to permit pivotal movement of the blade about the longitudinal axis of the blade post. Such pivotable movement facilitates manipulation of the blades with respect to the vertebral endplates to ease removal of blades and distractor.

US 6,739,068 B1 discloses pliers having a scale to read the amount of pivot between the separated jaws. Furthermore the pliers comprise a second scale for detecting the deformation of the lever arm to deduct the force applied to a work piece. The scales show present results of force and pivot value. It is stated that the use of a parallelogram construction allows that the location of the contact of the workpiece with the jaws is not critical to the readings. However, the value of the exerted force heavily depends on the position of the hand of the user on the handle.

US 4,899,761 A1 discloses a motor driven pliers device having force sensors to detect the force exerted on an object. The distance between the blades of the pliers are directly known from the position of the stepper motors opening the jaws. Said pliers device is not usable for manual interventions by a surgeon and are only intended to be used as checking device for experiments. Therefore the document discloses a possibility to save the measured values in an electronic storage device.

US 2006/0036258 A1 discloses different forms of the blades, especially oriented in an angle to the general longitudinal direction of the pliers. A measurement of the distances between the distraction prongs is compromised through the existing bending and torsion moments torques leading to some deformation of the pliers. Since there are no means to measure these forces, a compensation of this deformation is not possible. Furthermore there is no possibility to determine the stiffness

### Summary of the invention

The devices according to the prior art do not allow to demonstrate the stiffness of the bone segment being engaged by the blades of the pliers. Furthermore, beside a device only usable in experimental environments there is no possibility to save the values as such or to memorize maximum values.

It is one object of the invention to provide manually usable pliers for separating two adjacent bone segments, i.e. a device which can be used by a surgeon in surgical applications, wherein said pliers have the possibility to measure the force exerted on said bone segments, the distance between the bone segments as well as the stiffness of said adjacent bone segments.

It is a further object of the invention to provide manually usable pliers which can be used in a more versatile manner.

It is a still further object of the invention to provide a surgeon with a direct feedback on the exerted force and the stiffness of the engaged bone segments.

The surgical instrument according to the invention allows the simultaneous measurement of force and distance using integrated sensors. The values can be shown on a display in real-time and they can be saved for later reference.

Advantageous embodiments use features as described in the dependent claims.

Especially, the invention allows to disregard the position of the surgeon's hand on the handle. Said position has no influence on the measurement any more. The features of the invention allows correct measurement with a manually used instrument by contrast to the device disclosed in US 4'899'761.

The present invention has several advantages over prior art, allowing correct measurement of stiffness of the engaged bone segments, which is due to the arrangement of the sensors supported by the calculations reducing the disturbances to a minimum and/or compensating them. This is achieved through using sensors arranged to measure the twisting or torsional moment as well as the bending moment and this can even be achieved with different blades and engagement prongs.

The integration of the sensors allows the reduction of size of the device and it can be sterilized easily.

Although the application shown in the drawings relate to vertebrae, the distractor can be equally be used with other bone segments which have to be separated. Such bone segments can be smaller and less resistant against distraction than the separation of two vertebrae.

A further advantage of the invention is the use of wireless transmission techniques to read out the measurements. This improves the usability of the device for the surgeon.

### Short Description of the drawings

The invention is now described in connection with advantageous embodiments shown in the enclosed drawings:

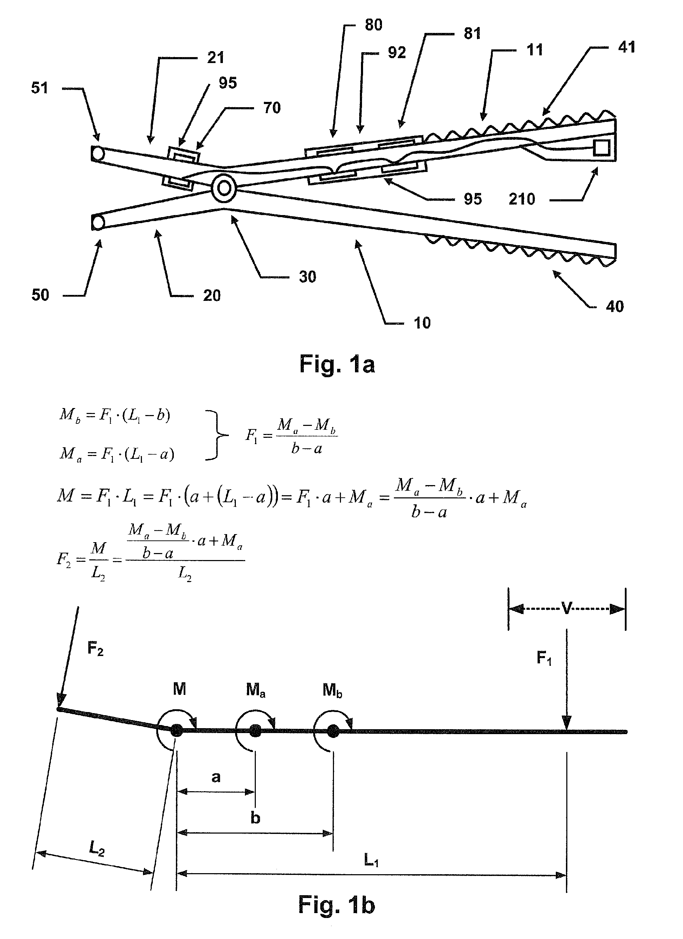

- Fig. 1 a: shows a schematic side view of pliers according to a first embodiment of the invention having a distance sensor and two sensors to sense bending moments wherein both sensors to sense bending moments are arranged along one handle between the fulcrum and the handle zone.
- Fig. 1 b: shows the formulas and the relationship between the forces and bending moments in the embodiment according to Fig. 1a.
- Fig. 2a: shows a schematic side view of pliers according to a second embodiment of the invention having a distance sensor and one sensor to sense bending moments wherein said last mentioned sensor is arranged between the fulcrum and the blades.
- Fig. 2b: shows the formulas and the relationship between the forces and bending moments in the embodiment according to Fig. 2a.
- Fig. 3a: shows a schematic side view of pliers according to a third embodiment of the invention having a distance sensor and one sensor to sense bending moments wherein said last mentioned sensor is arranged in the axis of the fulcrum.
- Fig. 3b: shows the formulas and the relationship between the the forces and bending moments in the embodiment according to Fig. 3a.
- Fig. 4a: shows a three dimensional view of a further modified embodiment of pliers having angled blades with a torsion sensor in the jaw area.
- Fig. 4b: shows the formulas and the relationship between the the forces and bending moments in the embodiment according to Fig. 4a.
- Fig. 5a: shows a side view of a pair of blades in an open state.
- Fig. 5b: shows a sectional view of the mount of the blades.
- Fig. 5c: shows a front view of a pair of blades having complementary V profiles.
- Fig. 5d: shows a front view of a pair of blades having complementary U/T profiles.

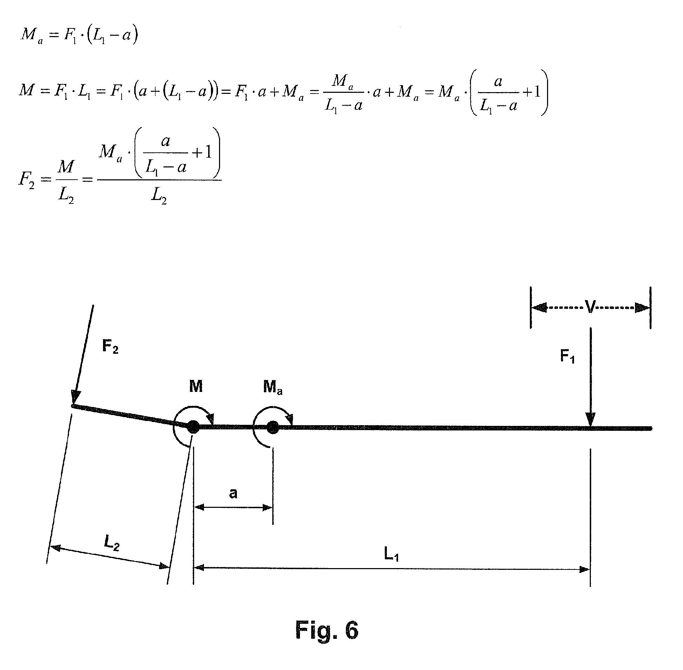

- Fig. 6: shows the formulas and the relationship between the the forces and bending moments according to prior art arrangements.
- Fig. 7a: shows an example of an embodiment with a wireless RF connection between pliers according to the invention and a control unit.
- Fig. 7b: shows a block diagram relating to Fig. 7a.
- Fig. 8a: shows a further example of an embodiment with a cable connection between pliers according to the invention and a control unit.
- Fig. 8b: shows a block diagram relating to Fig. 8a.
- Fig. 9a: shows a schematic perspective view of pliers according to a fourth embodiment introducing a web element having integrated sensors.
- Fig. 9b: shows the arrangement of a combined bridge sensor adapted for simultaneous measurement of bending and torsional moments.
- Fig. 9c: shows the formulas and the relationship between the the forces and bending moments in a combined bridge sensor according to Fig. 9b.
- Fig. 9d: shows method steps of calculation of errors of measurement.
- Fig. 10a: shows a schematic side view of the pliers in a closed state when used between two adjacent vertebrae.
- Fig. 10b: shows a schematic side view of the pliers in an opened state when used between two adjacent vertebrae.
- Fig. 10c: shows a view from above onto the pliers when used between two adjacent vertebrae in a vertical relationship to the pivot axis.

### Exemplary description of advantageous embodiments

Intelligent surgical instruments using integrated sensors permit the surgeon to make a so-called local navigation using real-time measurements of distance, forces and torsions improving especially the efficiency of minimal invasive interventions.

Fig. 1 a shows a schematic side view of pliers according to a first embodiment of the invention having a displacement or distance sensor 70 and two sensors 80 and 81 to sense bending moments wherein both sensors 80, 81 to sense bending moments are arranged along one of the two handle parts, here handle part 11, between the fulcrum 30 and the handle gripping zone 40, 41. The distance sensor 70 can be positioned in the handle area 10, 11 as well as in the jaw area 20, 21.

The distance sensor 70 is preferably an optical or magnetic sensor as explained below. The two sensors 80 and 81 can be realized through strain gauges. A strain gauge is a device used to measure deformation of an object.

Fig. 1 b shows the formulas and the relationship between the the forces and bending moments in the embodiment according to Fig. 1a. F₁ is the force applied by the surgeon on the pliers through gripping the handles 10, 11 in the handle gripping zone 40, 41. F₂ is the force, the adjacent bone segments exert onto the pliers and the blades 50, 51. M_{a,b} are the measured bending moments. M is the bending moment to calculate the force F₂ since the distance L₂ is known. V defines the area of the variable position for application of force F₁, i.e. V can be extended between the entire gripping zone 40, 41.

The pliers comprise the two handle parts 10, 11 as well as the two jaws 20, 21 which can be pivoted around the common fulcrum 30. Handles 10, 11 and jaws 20, 21 are mounted to execute an opposite movement. The jaws 20, 21 comprise bone engagement ends 50 and 51. The displacement sensor 70 measures the distance between the two handles (or jaws) depending on the position where the sensor 70 is mounted. This distance value can be used to evaluate the current opening angle between the two jaws.

In case of an optical sensor 70 for the distance measurement, a light beam is sent from the sensor 70 on jaw 21 towards a reflection area 71 on the opposite jaw 20. Through reflection of the beam from area 71 back and e.g. using triangulation, the distance can be calculated. The area 71 usually does not need a special treatment in view of the reflection properties of the materials used for surgical instruments.

If the pliers are made from magnetic material, a magnetic sensor 70 can be used in a similar way. Then the position of area 71 in opposite jaw 20 depending on the opening angle of the pliers influences the magnetic field detected by the magnetic sensor 70. If the pliers are not magnetic then a small magnetic insert in the region of area 71 in jaw 20 allows the detection of the opening angle of the jaws.

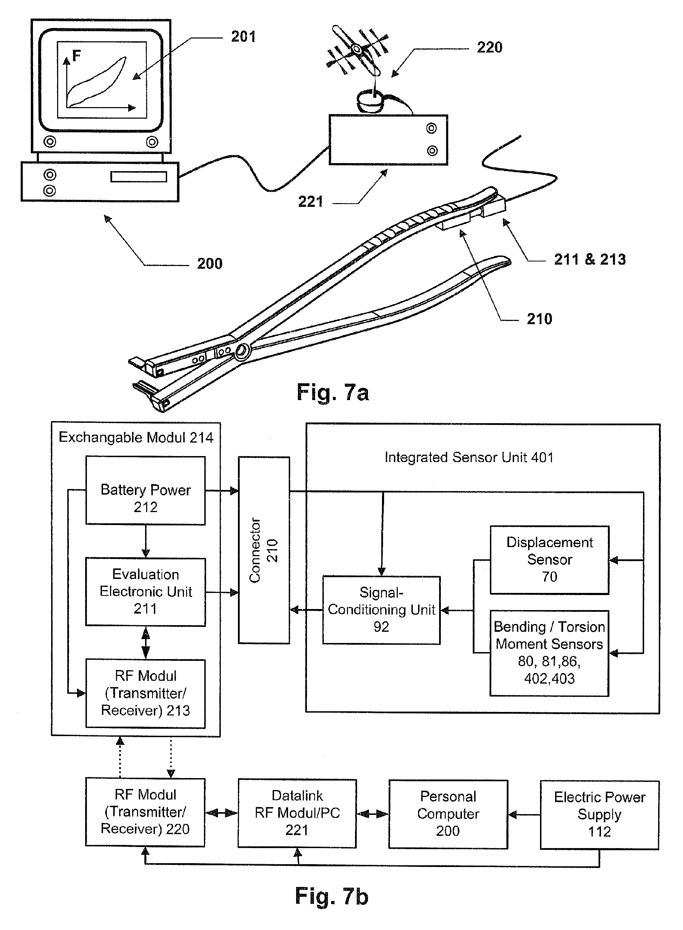

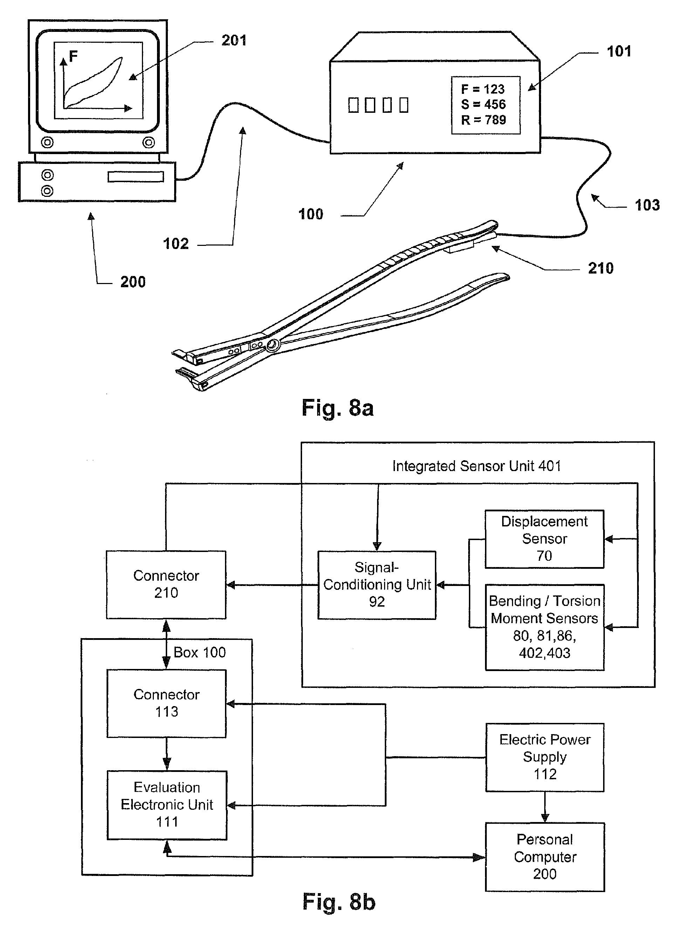

According to the embodiment shown in Fig. 1 a the two sensors detecting bending moments are positioned within or along the handle parts 10, 11, and this between the fulcrum 30 and the handle gripping zones 40, 41, preferably near the fulcrum 30. Such pliers can be used in conjunction with further equipment according to Fig. 7 or 8.

The formulas according to Fig. 1 b can be explained as follows: In order to calculate the force F₂, bending moment M and distance L₂ have to be known. L₂ represents a fixed distance and can therefore me measured once and then registered. The moment M itself is not measured directly but calculated based on the knowledge of the bending moments Mₐ and M_{b}. Therefore F₁ and distance L₁ have to be known for calculation of M. The two variables F₁ and L₁ can be calculated using two sensors 80, 81 (Mₐ and M_{b}). for measurements of signals. These bending moments sensors allow the preparation of two equations to be solved to obtain the two then unknown values.

The sensors 70, 80 and 81 as well as the signal treatment unit 92 are cast in a thermoresistant polymer 95 and connected to the connector 210 with heat resistant electric wire 96 firmly inserted or attached to the body of the pliers. If such pliers are used in conjunction with a device according to Fig. 7a/b, then the evaluation electronic unit 211, the electric power supply 212 and the transmitter/receiver 213 can be mounted as an exchangeable module 214 in one of the handle parts 40 or 41. The electric connection with the sensors can be realized using corresponding multiple pole male / female type connectors 210. The data transmission uses a wireless RF connection with an external transceiver 220, being connected to a personal computer 200 via a datalink 221. Display 201 of the values and storage of said values is performed on said PC 200. The power supply 212 can be a battery or a supercap. The pliers having the connector 210 can readily be sterilised as a conventional surgical instrument. The exchangeable module 214 can be removed beforehand. Such a connection can be WiFi, Bluetooth or any other preferably local wireless protocol.

According to a another device for signal transmission according to Fig. 8a, 8b, the evaluation electronic unit 111 are provided in an external box 100. This box can perform the display 101 and the storage of the values or which may be connected using a data link 102 with a personal computer 200. The connection between pliers and box is realized using a corresponding cable 103 for data transfer and which is connected with the connector 210 at the pliers. The box 100 can also be integrated as software instructions into the computer 200. An external medical grade power supply 112 supplies computer 200, box 100 and the pliers. The pliers having the connector 210 can readily be sterilised as a conventional surgical instrument. The box 100 and computer 200 do not need to undergo such a treatment. The cable 103, if necessary, can also be sterilised.

In both cases, according to Fig. 7 or according to Fig. 8, the sensor element is encapsulated in a sterilisable way in or onto the pliers. This is especially true for the wire between the connector 210 and the sensor element or sensor elements 70, 80 and 81. The replaceable sensor element 82 can be mounted in the pliers and covered with a protective surface allowing for different sterilization techniques.

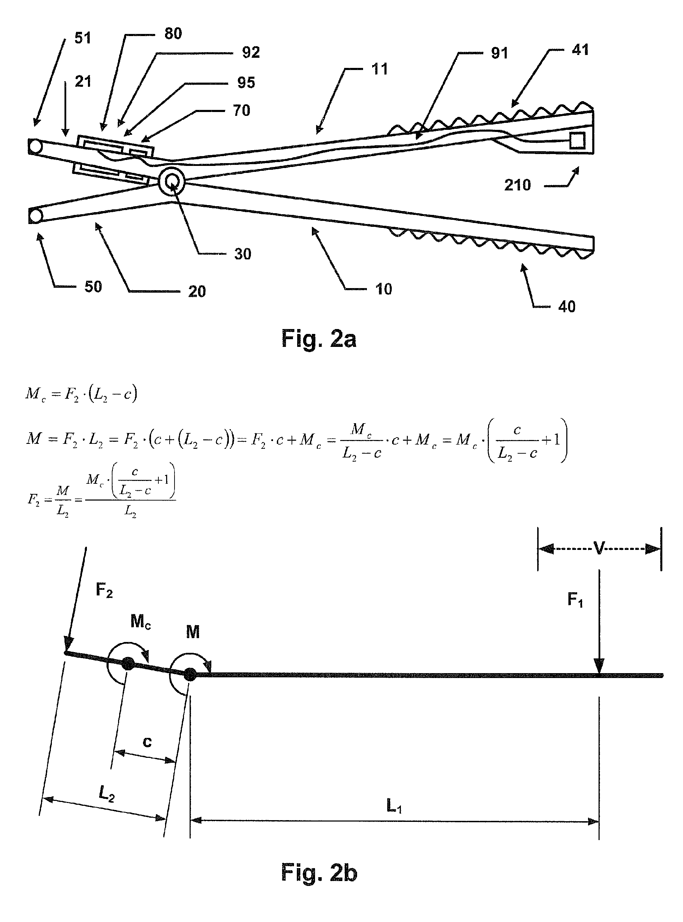

Fig. 2a shows a schematic side view of pliers according to a second embodiment of the invention having a distance sensor 70 and one sensor 80 to sense bending moments wherein said last mentioned sensor is arranged between the fulcrum 30 and the bone engagement ends or blades 50, 51. The distance sensor 70 can be positioned in the handle area 10, 11 as well as in the jaw area 20, 21. The pliers comprise in the jaw 21 only one displacement sensor 70 and only one bending sensor 80, which are both arranged between the fulcrum 30 and the bone engagement ends 50, 51. The pliers according to this embodiment can be used in connection with devices according to Fig. 7 or 8.

F₁ and F₂ are the same forces as defined above. M_{c} is the measured bending moment. M is the bending moment to calculate the force F₂. V defines the area of the variable position for application of force F₁ as above.

The following description of the pliers can be applied to all embodiments shown, especially the embodiments shown in Fig. 1, 2, 3 and 9, i.e. same features receive identical reference numerals.

The formulas according to Fig. 2b are used to calculate the force F₂, while the moment M and the distance L₂ are to be known. L₂ is a known fix distance and is determined in advance. M is not measured but is to be calculated based on the moment M_{c}, since distance c is known. Distance c is fix and can be determined once in advance.

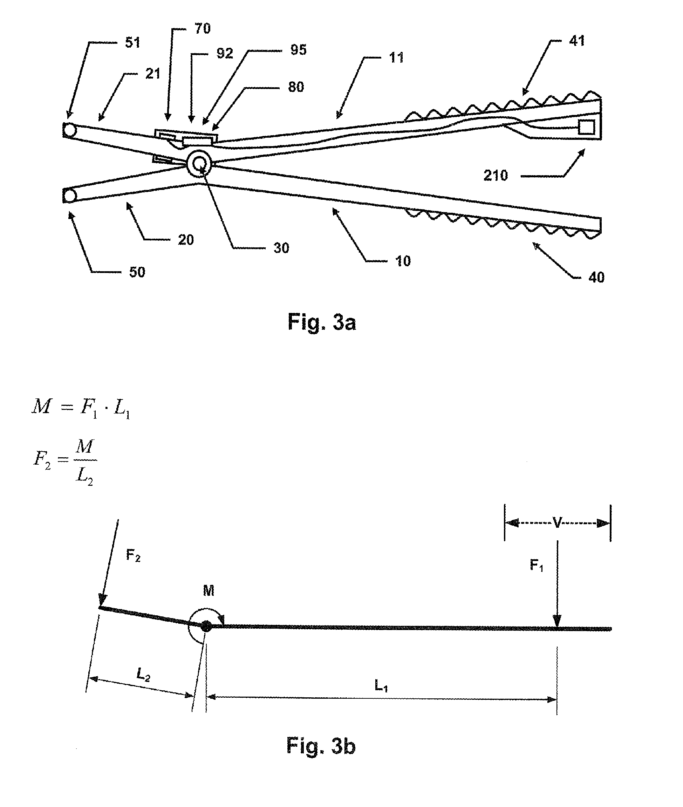

Fig. 3a shows a schematic side view of pliers according to a third embodiment of the invention having a distance sensor 70 and one sensor 80 to sense bending moments wherein said last mentioned sensor is arranged in the axis of the fulcrum 30. The distance sensor 70 can be positioned in the handle area 10, 11 as well as in the jaw area 20, 21. The pliers according to this embodiment can be used in connection with devices according to Fig. 7 or 8.

The formulas according to Fig. 3b are used to calculate the force F₂, while the moment M and the distance L₂ are to be known. L₂ is a known fix distance and is determined in advance. M is measured using the sensor 80. F₁, F₂ and V are defined as above. M represents the bending moment which is measured and needed to calculate the stiffness of the adjacent bone elements. The stiffness can be calculated as the quotient of an infinitesimal change of the force value exerted by the surgeon against the corresponding infinitesimal change of the distance between the distracted bone segments. These considerations relating to the evaluation of stiffness are valid for all embodiments throughout this description.

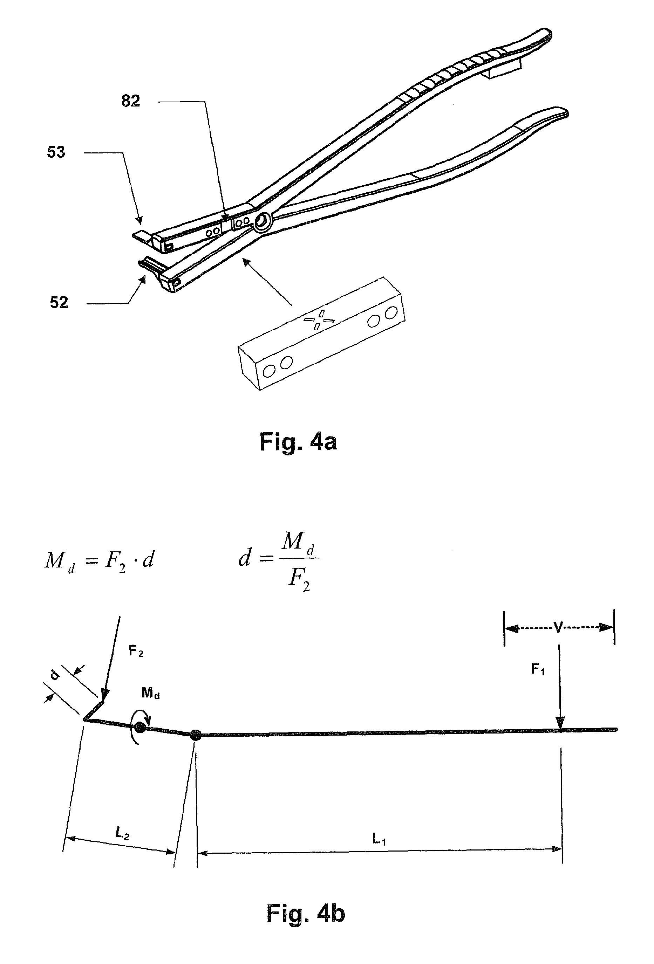

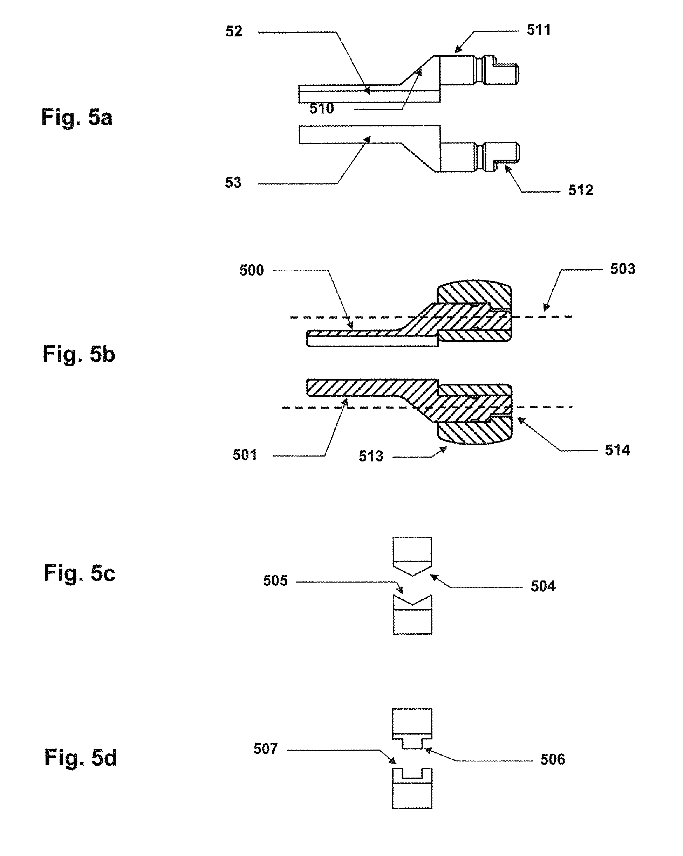

Fig. 4a shows a three dimensional view of a further modified embodiment of pliers having bone engagement ends with angled blades 52, 53 with a sensor unit 82 to acquire torsional moments in the jaw area. The torsional sensor shown in fig.4a is comprised of a sensor support block 401 and a torsion sensor bridge 403. Fig. 4b shows the formulas and the relationship between the forces and torsion moments in the embodiment according to Fig. 4a. This torsion measurement is preferably used in combination with an embodiment of Fig. 1, 2 or 3, if the ends of the blades 52, 53 are laterally offset. Having the knowledge of the force F₂ applied at the ends 52, 53 the measured torsional moment allows to determine the point of application of the force against the contact surfaces 500, 501 as can be seen in Fig. 5b.

F₁ and F₂ are the same forces as defined above.. M_{d} is the measured torsional moment. V defines the area of the variable position for application of force F₁ as above. D is the lateral offset between the point of application of the Force F₂ and the longitudinal axis of the pliers.

The integrated sensor unit 82 may comprise the sensors 70 and 80 as mentioned in connection with Fig. 1 to 3. Sensor unit 82 according to Fig. 4a only shows a bridge 403 being a sensor for the measurement of torsion of the jaw 21.

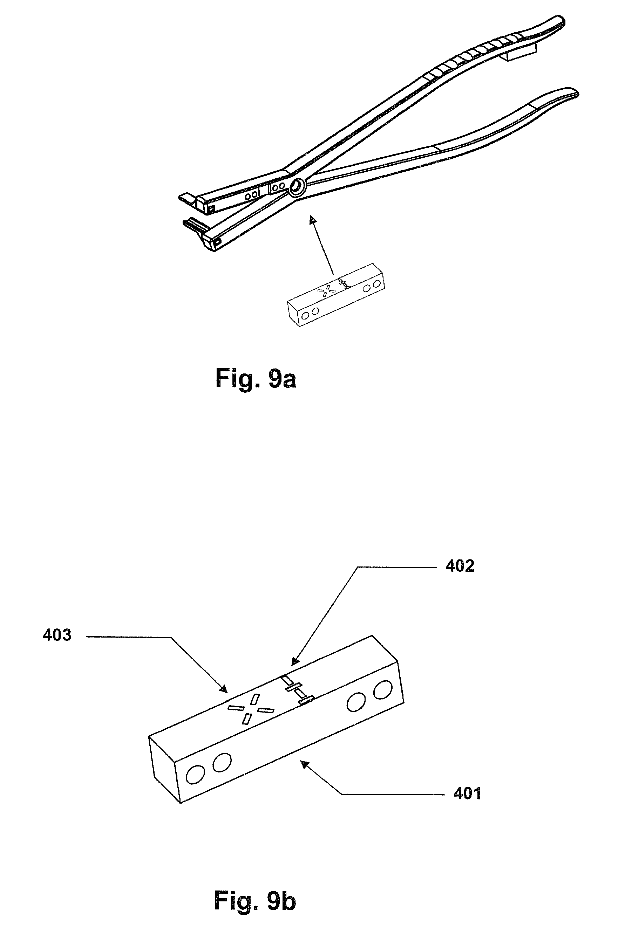

In connection with the embodiment of Fig. 4 said unit 82 should also comprise beside the sensor for the measurement of the torsion of the jaws related to the angled blade ends, which are not in the longitudinal direction of the pliers, a sensor for the bending of the jaws. A sensor unit showing such sensors can be seen from Fig. 9b. This torsion measurement is preferably used in combination with an embodiment of Fig. 1, 2 or 3 if the end of the blades 52, 53 are laterally offset. Having the knowledge of the force F₂ applied at the ends 52, 53 the measured torsional moment allows to determine the point of application of the force against the contact surfaces 500, 501 as can be seen in Fig. 5b.

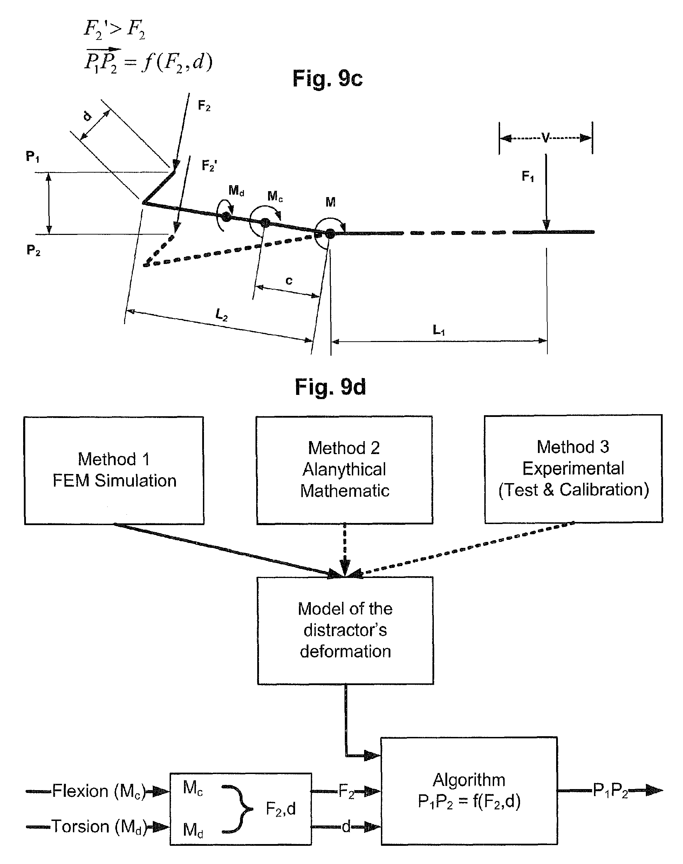

Fig. 5a shows a side view of a pair of bone engagement ends or blades in an open state and Fig. 5b shows a sectional view of the mount of the blades. The bone engagement ends with angled blades according to Fig. 5a to 5d may be used in connection with all embodiments as shown above, preferably with the fourth embodiment described in connection with Fig. 9. The angled blades 52, 53 can be rotated to some extent and are pivoted in the foremost end of the jaw area 20, 21 to allow and obtain a homogenous distribution of the pressure on the entire contact surface 500, 501.

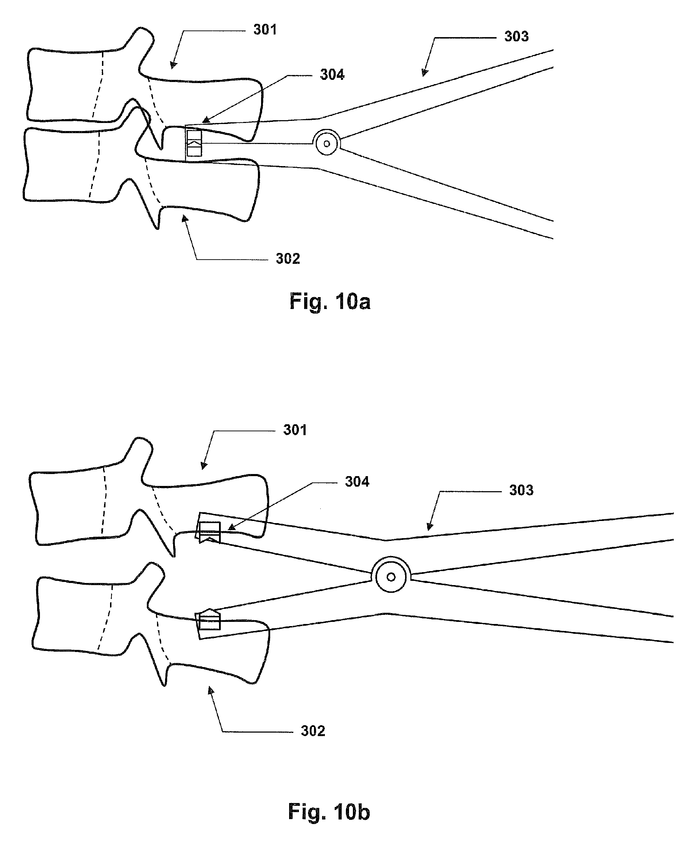

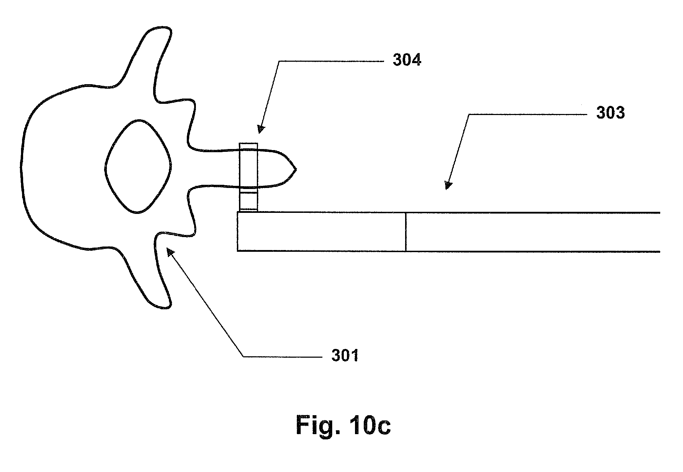

Fig. 10a shows a schematic side view of the pliers in a closed state when used between two adjacent vertebrae, wherein Fig. 10b shows a schematic side view of the pliers in the opened state. Fig. 10c shows a view from above onto the pliers in a vertical relationship to the pivot axis, when used in connection with blade ends according to Fig. 5.

A ring groove 510 in the mounting stud 511 ensures that the blades 52, 53 cannot get lost. The angle is limited through a flat portion 512 at the end of the studs 511. The flat portion is oriented to corresponding abutment surfaces 514. The contact surfaces 500, 501 between the angled ends 52, 53 and the bone segments 301, 302 (as seen in Fig. 10) are viewed in the direction of the force, offseted beyond the pivoting axis 503 of the foremost end 513 of the jaws 20, 21 to allow a stable configuration of the contact surfaces 500, 501. The geometry of the angled ends 52, 53 can be profiled as seen in Fig. 5c,d to improve their strength at minimum profile height. Such a height is interesting to allow introduction of the ends 52, 53 between bone segments separated only by a small distance.

The pivoting axis 503 can be oriented at 90° relating to the main longitudinal direction of the respective jaw 20 or 21. Although all drawings only show this right angle, It is also possible to have a different angle orientation between the blades 52, 53 and the jaws 20, 21, e.g. 60° or 30°.

Fig. 5c shows a front view of a pair of blades 504, 505 having complementary V profiles; the convex surface 504 can be centred through engagement with the concave surface 505. Fig. 5d shows a front view of a pair of blades 506, 507 having complementary U/T profiles, the T profile surface 506 can also be centred through engagement with the concave groove surface 507.

Fig. 9a shows a schematic perspective view of pliers according to a fourth embodiment introducing a removable sensor support block 401 showing the integrated sensors 402 and 403. Fig. 9b shows the arrangement of the combined bridge sensor 402, 403 adapted for simultaneous measurement of bending and torsional moments. Such a removable sensor support block 401 comprises the combination of a bending moment measurement, e.g. according to Fig. 2, together with a torsion measurement, e.g. according to Fig. 4, in combination with angled ends. Fig. 9c shows the formulas and the relationship between the forces, the bending moments and the torsional moments in a combined bridge sensor according to Fig. 9b. Preferably, the removable sensor support block 401 can be mounted in one of the jaws 20, 21. The removable sensor support block 401 uses a bending sensor bridge 402 to allow measurements of bending moments as well as a torsion sensor bridge 403 to measure torsional moments. Such torsional moments appear in combination with angled plier ends wherein the torsion of the jaws 20, 21 creates a measurement error when the position of the pliers ends 52, 53 are determined. Further measurement errors appear upon bending of the thin angled pliers ends and the bending of the jaws. Displacement sensor 70 and signal conditioning unit 92 are not visible in Fig. 9a or 9b, but can be positioned as shown in Fig. 1 a or fig. 2a or also integrated into the sensor support block 401.

Fig. 9d shows method steps of calculation of errors of measurement. Such method steps are prepared for each geometry of pliers. Someone skilled in the art can use different methods. A first method consists of modelling the geometry of the using finite element methods (FEM-program), defining the properties of the material and analysing the behaviour under load. A second method consists determining the mathematic formulas related to the distortion of the pliers under predetermined geometries and material in an analytical way. A third method uses the results of experiments. The behaviour of the pliers under load is monitored and therefore a relation between load and deformation can be obtained.

When the model is defined e.g. according to one of the described methods, a function f(F₂,d) can be defined calculating the bending vector P₁P₂ at the pliers ends, when the force F₂ and the point of application d is known. The measured values of the bending moment M_{c} and the torsion moment M_{d} are combined to calculate f(F₂,d). Therefore the measurement errors can be evaluated for a given geometry of the pliers when the bending and torsional moments are known.

The bone engagement ends 50 and 51 used in connection with the embodiments of Fig. 1 to 4 and 9 can be shaped as shown in Fig. 5. However, they can also be oriented in the direction of the main longitudinal direction of the pliers. They can also be releasably connected with a mounting stud as the bone engagement ends 52 and 53 according to Fig. 5, but the opening to accommodate such a connection stud of a bone engagement end 50 or 51 is then oriented in said main longitudinal direction of the pliers. Within such an embodiment the stud is preferably not rotatable around said axis, but it can still be replaced according to the needs of the surgeon.

In all these cases of replacement of bone engagement ends 50, 51 or 52, 53, the calculation and calibration method to provide the compensation for specific bone engagement ends has to be chosen. Of course such adapted values can already be prepared and stored in the control unit 100, 200 or 214.

### Reference numerals

- 10: handle
- 11: handle
- 20: jaw
- 21: jaw
- 30: fulcrum
- 50: bone engagement end
- 51: bone engagement end
- 52: bone engagement end, positioned at an angle with the jaw
- 53: bone engagement end, positioned at an angle with the jaw
- 70: displacement sensor
- 71: reflection area
- 80: bending moment sensor
- 81: bending moment sensor
- 82: torsion sensor
- 92: signal conditioning unit
- 95: thermoresistant polymer
- 96: electric wires
- 100: external box
- 101: display
- 102: datalink
- 103: cables for data transfer
- 111: evaluation electronic unit
- 112: electric power supply
- 113: connector
- 200: personal computer
- 201: display
- 210: connector
- 211: evaluation electronic unit
- 212: electric power supply
- 213: transmitter/receiver
- 214: exchangeable module
- 220: transceiver
- 221: datalink
- 301: bone segment
- 302: bone segment
- 303: handle
- 304: angled jaw end
- 401: sensor support block
- 402: bending sensor bridge
- 403: torsion sensor bridge
- 500: contact surface
- 501: contact surface
- 503: pivoting axis
- 504: V-shaped blade
- 505: V-shaped blade
- 506: T-shaped blade
- 507: groove surfaced blade
- 510: ring groove
- 511: mounting stud
- 512: flat portion
- 513: foremost end
- 514: abutment surfaces

## Claims

1. Manually usable pliers for separating two adjacent bone segments comprising handles (10, 11) connected with jaws (20, 21) through a fulcrum (30), **characterized in that** the pliers furthermore comprise a sensor (70) for determining the distraction distance between said two bone segments and at least one additional sensor (80, 81) for determining the distraction force acting on such bone segments upon opening of the jaws through manually applicable forces on the handle portions (40, 41), wherein the sensors (70, 80, 81, 82) are electronic sensors.

2. Pliers according to claim 1, **characterized in that** the additional sensors (80, 81, 82, 402, 403) are adapted to determine the bending and twisting under load of the jaws (20, 21) and/or of the handle portions (10, 11) or within the fulcrum (30), especially comprising strain gauges.

3. Pliers according to claim 1 or 2, **characterized in that** the sensor (70) for determining the distraction distance is a optical sensor, integrated in one jaw (21) or handle (11), generating a light beam directed on an opposite area (71) on the opposite (20) or handle (10) and adapted to receive a light beam reflected from said area (71) to calculate said distraction distance between said two bone segments based on the determined opening angle between the jaws (20, 21) or handles (10, 11) or that the sensor (70) for determining the distraction distance is a magnetic sensor, determining said opening angle based on the variation of the magnetic field sensed by the sensor due to magnetic material in the opposite jaw (20) or handle (10).

4. Pliers according to one of claims 1 to 3, **characterized in that** an electronic control unit is adapted to store the measured and calculated values of distance and force or derived values.

5. Pliers according to one of claims 1 to 4, **characterized in that** the at least one additional sensor (80, 81) for determining the distraction force acting on such bone segments comprise two force sensors for the determination of the bending moment along the handle (10, 11) between the fulcrum (30) and the handle gripping area (40, 41), preferably near the fulcrum (30).

6. Pliers according to one of claims 1 to 4, **characterized in that** the at least one additional sensor (80, 81) for determining the distraction force acting on such bone segments comprise one force sensor for the determination of the bending moment along one jaw (20, 21) between the fulcrum (30) and the bone engaging ends (50, 51, 52, 53), preferably near the fulcrum (30).

7. Pliers according to one of claims 1 to 4, **characterized in that** the at least one additional sensor (80, 81) for determining the distraction force acting on such bone segments comprise one force sensor for the determination of the bending moment being mounted at the fulcrum (30) between one jaw (20, 21) and one handle (10, 11).

8. Pliers according to one of claims 1 to 7, **characterized in that** a further sensor is provided in one jaw (20, 21) as a torsional sensor (82) between the fulcrum (30) and the bone engaging ends (50, 51, 52, 53), preferably near the fulcrum (30).

9. Pliers according to one of claims 1 to 8, **characterized in that** at least the sensor elements are covered with a protecting layer for protection against vapour sterilisation.

10. Pliers according to one of claims 1 to 9, **characterized in that** the sensors are provided in a removable sensor support block (401).

11. Pliers according to claim 10, **characterized in that** the pliers comprise a mounting place for the removable sensor support block (401) being a replaceable unit comprising any of the sensors (70, 80, 81, 82, 402, 403), especially comprising a signal conditioning unit (92).

12. Pliers according to claim 10 or claim 11, **characterized in that** the removable sensor support block (401) or any of the other sensors (70, 80, 81, 82, 402, 403) is connected with electric wires (96) to a connector (210) mounted on a handle (40, 41) of the pliers as an interface for external communication.

13. Pliers according to claim 12, **characterized in that** the pliers comprise a replaceable module having a wireless transceiver as an interface for external communication.

14. Apparatus for the determination of the stiffness of two adjacent bone segments while manually using pliers according to one of claims 11, 12 or 13, **characterized in that** the apparatus comprises an optical or acoustic display (201) and a complementary interface (103, 220) for external communication to communicate with the pliers for transmittal of measured and treated values for determining and display of stiffness values of said adjacent bone segments.

15. Apparatus according to claim 14, **characterized in that** the stiffness determination comprises the calculation of calibration values for a specific geometry of pliers, based on a modelling using a finite element method, defining the properties of the material and analysing the behaviour under load or based or determination of the mathematic formulas related to the distortion of the pliers under predetermined geometries and material in an analytical way and/or based on the results of experiments.

16. Manually usable pliers for separating two adjacent bone segments comprising handles (10, 11) connected with jaws (20, 21) through a fulcrum (30) having bone engagement ends (52, 53), which are releasably connected with the respective jaws (20, 21), **characterized in that** the jaws (20, 21) have lateral openings to accommodate a connection stud (511) of a bone engagement end (52, 53) oriented in an angle from the main longitudinal direction of the pliers.

17. Pliers according to claim 16, **characterized in that** the two bone engagement ends (52, 53) comprise complementary V-shaped profiles (504, 404) or U- and T-shaped profiles (506, 507).

18. Pliers according to claim 16 or 17, **characterized in that** the bone engagement ends comprise a ring groove (510) at the bearing surface.

19. Pliers according to one of claims 16 to 18, **characterized in that** the connection studs (511) of the bone engagement ends (52, 53) comprise a flattened surface (512) and **in that** the lateral openings comprise an abutment, especially a flattened, surface (514) to delimit the turning angle of the bone engagement ends (52, 53).

20. Pliers according to one of claims 16 to 18, **characterized in that** the connection studs (511) of the bone engagement ends (52, 53) are rounded allowing a complete turning angle of the bone engagement ends.

21. Pliers according to one of claims 16 to 18, **characterized in that** the angled bone engagement end (52, 53) comprise contact surfaces (500, 501) being positioned in direction of the forces on or beyond the pivot axis.

22. Pliers according to one of claims 1 to 15 and according to one of claims 16 to 21.
